(19) Europäisches Patentamt / European Patent Office / Office européen des brevets

(11) **EP 3 558 401 B1**

(12) # EUROPÄISCHE PATENTSCHRIFT

(45) Veröffentlichungstag und Bekanntmachung des Hinweises auf die Patenterteilung:
**07.12.2022 Patentblatt 2022/49**

(21) Anmeldenummer: **17826521.1**

(22) Anmeldetag: **22.12.2017**

(51) Internationale Patentklassifikation (IPC):
***A61L 26/00*** (2006.01)

(52) Gemeinsame Patentklassifikation (CPC):
(C-Sets verfügbar)
**A61L 26/0004; A61L 26/0019; A61L 26/0066; A61L 26/008; A61L 26/0095;** A61L 2300/104; A61L 2300/404; A61L 2300/406; A61L 2300/624

(Forts.)

(86) Internationale Anmeldenummer:
**PCT/EP2017/084410**

(87) Internationale Veröffentlichungsnummer:
**WO 2018/115453 (28.06.2018 Gazette 2018/26)**

(54) **WASSERHALTIGE HYDROGELZUSAMMENSETZUNG, UMFASSEND ELEMENTARE SILBERPARTIKEL**

WATER-CONTAINING HYDROGEL COMPOSITION, COMPRISING ELEMENTAL SILVER PARTICLES

COMPOSITION AQUEUSE D'HYDROGEL CONTENANT DES PARTICULES ÉLÉMENTAIRES D'ARGENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.12.2016 DE 102016125579**

(43) Veröffentlichungstag der Anmeldung:
**30.10.2019 Patentblatt 2019/44**

(73) Patentinhaber: **Paul Hartmann AG
89522 Heidenheim (DE)**

(72) Erfinder: **KETTEL, Markus
89522 Heidenheim (DE)**

(74) Vertreter: **Ter Meer Steinmeister & Partner
Patentanwälte mbB
Nymphenburger Straße 4
80335 München (DE)**

(56) Entgegenhaltungen:
WO-A1-00/78281      WO-A1-01/41819
WO-A1-2008/049527   WO-A2-2010/000450

• DAS ANUP ET AL: "Preparation and characterization of silver nanoparticle loaded amorphous hydrogel of carboxymethylcellulose for infected wounds", CARBOHYDRATE POLYMERS, APPLIED SCIENCE PUBLISHERS, LTD. BARKING, GB, Bd. 130, 7. April 2015 (2015-04-07), Seiten 254-261, XP029175019, ISSN: 0144-8617, DOI: 10.1016/J.CARBPOL.2015.03.082
• VICHAYARAT RATTANARUENGSRIKUL ET AL: "Development of Gelatin Hydrogel Pads as Antibacterial Wound Dressings", MACROMOLECULAR BIOSCIENCE, Bd. 9, Nr. 10, 8. Oktober 2009 (2009-10-08), Seiten 1004-1015, XP055464364, DE ISSN: 1616-5187, DOI: 10.1002/mabi.200900131
• G. Schneider (ras materials Gmbh): "Sol-gel Schichten aus Thüringen veredelt mit antimikrobiellem Nanosilber", , 1. Januar 2007 (2007-01-01), XP055464457, Germany ISBN: 978-3-921254-86-8 Gefunden im Internet: URL:http://www.thgot.de/wp-content/uploads /2015/09/Schneider-Gregor_16.09.15.pdf

(52) Gemeinsame Patentklassifikation (CPC): (Forts.)

C-Sets
**A61L 26/0019, C08L 75/04;**
**A61L 26/008, C08L 75/04**

## Beschreibung

Die Erfindung betrifft eine wasserhaltige Hydrogelzusammensetzung, umfassend elementare Silberpartikel sowie eine die Hydrogelzusammensetzung umfassende mehrschichtige Wundauflage. Die mehrschichtige Wundauflage wird insbesondere in der Inflammations- und/oder Granulationsphase zur Behandlung beispielsweise von Brandwunden und/oder chronischen Wunden eingesetzt. Ferner betrifft die Erfindung ein Verfahren zur Herstellung der Hydrogelzusammensetzung.

## Hintergrund der Erfindung

Unter einer Wunde wird in der Literatur die Trennung des Zusammenhangs von Geweben der Körperhülle bei Menschen oder Tieren verstanden. Sie kann mit einem Verlust an Substanz verbunden sein. Die Heilung von Hautwunden beruht auf der Fähigkeit der Haut, Epithel- sowie Binde- und Stützgewebe zu regenerieren. Die Regeneration selbst ist durch ein komplexes Geschehen von ineinander übergreifenden Zellaktivitäten gekennzeichnet, die den Heilungsprozess schrittweise vorantreiben. So werden in der Literatur unabhängig von der Art der Wunde drei wesentliche Wundheilungsphasen beschrieben. Hierzu gehört die inflammatorische oder exsudative Phase zur Blutstillung und Wundreinigung (Phase 1, Inflammationsphase), die proliferative Phase zum Aufbau von Granulationsgewebe (Phase 2, Granulationsphase) und die Differenzierungsphase zur Epithelisierung und Narbenbildung (Phase 3, Epithelisierungsphase).

Zur Unterstützung der einzelnen Wundheilungsphasen ist die Verwendung von Wundauflagen von großer Bedeutung. So wird beispielsweise in der WO 2010/000451 eine mehrschichtige Wundauflage mit einer Hydrogelmatrix sowie in der WO 2011/141454 eine Wundauflage zur Wundbehandlung mit einem superabsorbierenden Material beschrieben, die sich jeweils insbesondere dafür eignen, die Wunde in einem feuchten Milieu zu halten. Die WO 2010/000451 beschreibt insbesondere Hydrogele zur Hydrotherapie von Wunden.

Zur Vorbeugung und Behandlung von Wundinfektionen sind im Stand der Technik Wundauflagen von Bedeutung, welche zusätzlich antimikrobielle Eigenschaften aufweisen.

Elementares Silber wird seit vielen Jahren erfolgreich als antimikrobielles Mittel in der Wundbehandlung eingesetzt. Aufgrund des elektrochemischen Potenzials von elementarem Silber werden jeweils nur geringe Mengen von Silberionen freigesetzt, die effektiv Bakterien hemmen oder töten können. Ein Vorteil von Silberionen besteht insbesondere darin, dass bisher keine oder kaum Resistenzen in Bakterien nachgewiesen worden sind. Dabei ist zu beachten, dass eine Erhöhung der freigesetzten Silberionenmenge zwar für die antimikrobielle Aktivität vorteilhaft ist, sich dadurch jedoch das Risiko einer unerwünschten Aufnahme von Silber in den menschlichen Organismus erhöht. Aufgenommenes Silber kann vom Körper nicht eliminiert werden, sondern wird in Form von elementarem Silber ins Gewebe eingelagert, was im Extremfall zu irreversiblen Hautverfärbungen (Argyrose bzw. Argyrie) führen kann.

Das Anup et al. beschreiben antimikrobielle Carboxymethylcellulose-Hydrogele die nanopartikuläres Silber enthalten [CARBOHYDRATE POLYMERS, APPLIED SCIENCE PUBLISHERS, LTD. BARKING, GB, Bd. 130, 07.04.2015, 254-261, XP029175019]

## Aufgabe und kurze Darstellung der Erfindung

Eine Aufgabe der vorliegenden Erfindung bestand deshalb darin, eine silberhaltige antimikrobielle Zusammensetzung zu finden, die eine ausreichend hohe Silberionenfreisetzung ermöglicht, um während der Wundbehandlung eine effektive antimikrobielle Aktivität zu gewährleisten, dabei jedoch die Freisetzung von Silberpartikeln und deren Aufnahme in den menschlichen Körper wirksam verhindert.

Es soll somit mit der vorliegenden Erfindung ein System zur Wundtherapie zur Verfügung gestellt werden, mit dem eine Wundbehandlung möglichst wirksam durchgeführt werden kann und welches eine hohe antimikrobielle Aktivität aufweist. Ferner soll die Wundheilung gefördert und beschleunigt werden. Zusätzlich soll eine Wundauflage bereitgestellt werden, welche einen hohen Tragekomfort bietet und selbst bei langer Tragedauer eine effektive antimikrobielle Wirksamkeit aufweist.

Die Aufgabe wurde überraschend gelöst durch die Bereitstellung einer Hydrogelzusammensetzung gemäß Anspruch 1, umfassend elementare Silberpartikel, eine die Hydrogelzusammensetzung umfassende mehrschichtige Wundauflage gemäß Anspruch 7 sowie ein Verfahren zur Herstellung der Hydrogelzusammensetzung gemäß Anspruch 12, wie hierin beschrieben.

Ein Gegenstand der Erfindung ist daher eine wasserhaltige Hydrogelzusammensetzung, umfassend elementare Silberpartikel mit einem durchschnittlichen Partikeldurchmesser, bestimmt durch Transmissionselektronenmikroskopie (TEM), von 5-20 nm, und einer Partikelgrößenverteilung, bestimmt durch Laserdiffraktometrie, von D90 ≤ 25 nm, wobei der Silbergehalt, bezogen auf das Gesamtgewicht der Hydrogelzusammensetzung, zwischen 15 und 500 ppm liegt, wobei die Hydrogelzusammensetzung ferner ein nichtionisches Tensid umfasst, wobei die Hydrogelzusammensetzung ein Polyurethan-Polyharnstoff-Copolymer umfasst.

Ein weiterer Gegenstand der Erfindung besteht in der Bereitstellung einer mehrschichtigen Wundauflage, umfassend mindestens eine wasserundurchlässige und wasserdampfdurchlässige Trägerschicht, eine absorbierende Schicht sowie eine die wasserhaltige Hydrogelzusammensetzung gemäß der vorliegenden Erfindung umfassende Schicht.

Ein weiterer Gegenstand der Erfindung besteht in der Bereitstellung eines Verfahrens zur Herstellung einer Hydrogelzusammensetzung, umfassend das Umsetzen einer Mischung, enthaltend:

(a) ein Polyamin,
(b) ein nichtionisches Tensid,
(c) optional einen mehrwertigen Alkohols ausgewählt aus Propylenglycol und/oder Glycerin,
(d) elementare Silberpartikel mit einem durchschnittlichen Partikeldurchmesser, bestimmt durch Transmissionselektronenmikroskopie (TEM), von 5-20 nm und einer Partikelgrößenverteilung, bestimmt durch Laserdiffraktometrie, von D90 ≤ 25 nm, und
(e) Wasser,

mit einem aliphatischen Diisocyanatpolymer, um eine wasserhaltige Hydrogelzusammensetzung zu bilden, welche ein Polyurethan-Polyharnstoff-Copolymer umfasst, wobei der Silbergehalt, bezogen auf das Gesamtgewicht der Hydrogelzusammensetzung, zwischen 15 und 500 ppm liegt.

## Detaillierte Beschreibung der Erfindung

Es konnte in der vorliegenden Erfindung überraschend gezeigt werden, dass eine hohe antimikrobielle Wirkung erzielt werden kann, wenn die Silberpartikel in einem erfindungsgemäßen

Hydrogel enthalten sind, und das Hydrogel einen Silbergehalt zwischen 15 und 500 ppm (bzw. 0,0015 bis 0,05% w/w) aufweist, wobei die Silberpartikel einen durchschnittlichen Partikeldurchmesser von 5-20 nm sowie eine Partikelgrößenverteilung von D90 ≤ 25 nm aufweisen.

Der Begriff "*Hydrogelzusammensetzung*" wird im Folgenden auch synonym als "*Hydrogel*" oder "*Hydrogelmatrix*" bezeichnet.

In der Hydrogelzusammensetzung der vorliegenden Erfindung werden die Vorteile einer hydroaktiven Wundauflage, die Wundexsudat absorbieren kann und keine traumatischen Effekte auf der Wunde hat, mit den antimikrobiellen Eigenschaften von Silbernanopartikeln kombiniert. Insbesondere wird hierbei elementares Nanosilber homogen in die üblicherweise quervernetzte Struktur eines Hydrogels eingeschlossen.

Bei dem Begriff "*Nanosilber*" handelt es sich um elementare Silberpartikel mit einem durchschnittlichen Partikeldurchmesser von 5-20 nm, bevorzugt von 9-18 nm. Der Partikeldurchmesser kann beispielsweise durch Transmissionselektronenmikroskopie (TEM) bestimmt werden.

Die Silberpartikel weisen eine enge numerische Partikelgrößenverteilung von D90 ≤ 25 nm auf, bevorzugt von D90 ≤ 20 nm, besonders bevorzugt von D90 ≤ 18 nm. Insbesondere beträgt die Partikelgrößenverteilung D99 ≤ 25 nm, bevorzugt D99 ≤ 20 nm, besonders bevorzugt D99 ≤ 18 nm. Der D90 (bzw. D99)-Wert gibt an, dass 90% (bzw. 99%) der Partikel kleiner sind als der angegebene Wert. Die Partikelgrößenverteilung kann beispielsweise durch Laserdiffraktometrie bestimmt werden.

Derartige elementare Silberpartikel sind kommerziell erhältlich als agpure® W10 (RAS materials GmbH, Regensburg, Deutschland) in Form einer wässrigen Nanosilberdispersion mit einem Anteil von 10 Gew.-% (w/w) Silber. Die Dispersion enthält zusätzlich <10 Gew.-% Tween 20 und Polysorbitol als stabilisierende Agentien.

Der Silbergehalt der Hydrogelzusammensetzung, bezogen auf das Gesamtgewicht der Hydrogelzusammensetzung, beträgt zwischen 15 und 500 ppm, bevorzugt zwischen 25 und 250 ppm, besonders bevorzugt zwischen 75 und 200 ppm.

Die antimikrobielle Wirksamkeit der silberhaltigen Hydrogelzusammensetzung der Erfindung wird ermöglicht durch kontrollierte Silberionenfreisetzung aus dem Hydrogel bedingt durch Oxidation und Ionentransport durch das wässrige System des Hydrogels. Es kann hierbei eine gesteuerte Oxidation der elementaren Nanosilberpartikel über vorhandenen bzw. transportierten Sauerstoff in der wässrigen Phase zu den elementaren Silbernanopartikeln erfolgen. Der gesteuerte Silberionentransport erfolgt insbesondere durch die wässrige Phase des Hydrogels in die wässrige bzw. exsudative Umgebung. Hierdurch wird eine kontrollierbare Silberionenfreisetzung erreicht, welche eine hohe antimikrobielle Wirksamkeit bereits bei geringer Nanosilberkonzentration im Hydrogel ermöglicht.

Bevorzugt werden mindestens 3 log Stufen an Reduktion der Keime erreicht, wie beispielsweise durch einen Hemmhoftest bestimmbar.

Aufgrund der geringen Freisetzung an Silberpartikeln sind die Hydrogelzusammensetzungen der vorliegenden Erfindung gegenüber Humanzellen nicht zelltoxisch und verursachen üblicherweise keine allergischen Reaktionen.

Der Wassergehalt der erfindungsgemäßen Hydrogelzusammensetzung beträgt vorzugsweise 20 bis 90 Gew.-

%, insbesondere 30 bis 85 Gew.-%, insbesondere 40 bis 80 Gew.-% und ganz besonders bevorzugt 50 bis 75 Gew.-% Wasser. Somit kann eine Wundauflage bereitgestellt werden, die eine für eine natürliche Wundheilung ausreichende Menge Feuchtigkeit zur Verfügung stellt.

Als wasserhaltige Hydrogelzusammensetzungen können im Zusammenhang mit der vorliegenden Erfindung insbesondere Zusammensetzungen verwendet werden, die eine zusammenhängende, diskrete Schicht bilden und unter Druck kein Wasser abgeben.

Bevorzugt kann die Hydrogelzusammensetzung einen hydrophilen Polyurethanschaum umfassen. Als Polyurethanschaum kann im Zusammenhang mit der vorliegenden Erfindung jeder heute in der modernen Wundbehandlung übliche hydrophile Polyurethanschaum verwendet werden, der einen Wasseranteil in seine Polyurethanmatrix aufnimmt und eine ausreichende Absorption aufweist. Damit ist im Zusammenhang mit der vorliegenden Erfindung unter einem hydrophilen Polyurethanschaum ein solcher Polyurethanschaum zu verstehen, der eine Flüssigkeit in seine Polyurethanmatrix und in seine Poren aufnehmen und speichern, somit absorbieren, kann und zumindest einen Teil der aufgenommenen Flüssigkeit wieder abgeben kann. Als hydrophile Polymerschäume sind hierbei insbesondere offenporige, hydrophile Polyurethanschäume geeignet.

Alternativ oder zusätzlich kann das Hydrogel auch ein superabsorbierendes Material, bevorzugt ein anionisches, superabsorbierendes Material, üblicherweise ein Polymer, insbesondere ein zumindest partiell quervernetztes Polymer, beispielsweise auf Polyacrylatbasis umfassen.

Erfindungsgemäß umfassen die Hydrogelzusammensetzungen ein Polyurethan-Polyharnstoff-Copolymer. Dieses Polyurethan-Polyharnstoff-Copolymer kann dabei insbesondere aus einem Präpolymer mit aliphatischen Diisocyanat-Gruppen und einem Polyamin auf Polyethylenoxidbasis gebildet werden. Insbesondere kann das Polyurethan-Polyharnstoff-Copolymer dabei aus einem Präpolymer mit Isophorondiisocyanat-Enden, einem Polyamin auf Polyethylenoxidbasis und Wasser gebildet werden. Diese Hydrogelzusammensetzungen sind besonders gut geeignet, Wasser einzulagern und dieses Wasser an eine Wunde abzugeben.

Weiterhin bevorzugt kann die wasserhaltige Hydrogelzusammensetzung weiterhin mindestens einen mehrwertigen Alkohol aus der Gruppe der zweiwertigen, dreiwertigen, vierwertigen, fünfwertigen oder sechswertigen Alkohole umfassen. Insbesondere kann der Alkohol ausgewählt werden aus der Gruppe der Glykole, insbesondere Ethylenglycol oder Propylenglycol, sowie Sorbitol oder Glycerin oder Mischungen hiervon. Diese Alkohole sind hervorragend als Feuchtigkeitsspender geeignet und stellen somit für die die Wunde umgebende Haut eine pflegende Komponente dar.

Dabei kann die wasserhaltige Hydrogelzusammensetzung insbesondere 0 bis 50 Gew.-% eines mehrwertigen Alkohols umfassen. Insbesondere umfasst die Hydrogelzusammensetzung 5 bis 40 Gew.-% eines mehrwertigen Alkohols und ganz besonders bevorzugt 10 bis 30 Gew.-% eines mehrwertigen Alkohols. Besonders bevorzugt enthält die Hydrogelzusammensetzung einen mehrwertigen Alkohol, ausgewählt aus Propylenglycol und/oder Glycerin, bevorzugt Glycerin. Der Anteil der Hydrogelzusammensetzung an Propylenglycol und/oder Glycerin beträgt insbesondere 5 bis 30 Gew.-%, bezogen auf das Gesamtgewicht der Hydrogelzusammensetzung, bevorzugt 10 bis 25 Gew.-%, besonders bevorzugt 15 bis 20 Gew.-%.

Insgesamt kann gemäß der vorliegenden Erfindung vorgesehen sein, dass die wasserhaltige Hydrogelzusammensetzung mindestens 10 Gew.-%, bevorzugt mindestens 15 Gew.-% Polyurethan-Polyharnstoff-Copolymer umfasst. Hierbei kann weiterhin bevorzugt vorgesehen sein, dass die Hydrogelzusammensetzung aus 6 bis 60 Gew.-% eines Präpolymers mit aliphatischen Diisocyanat-Gruppen, 4 bis 40 Gew.-% Polyamin auf Polyethylenoxidbasis, 0 bis 50 Gew.-% eines mehrwertigen Alkohols und mindestens 20 Gew.-% Wasser gebildet wird.

Weiterhin bevorzugt kann vorgesehen sein, dass die Hydrogelzusammensetzung aus 6 bis 30 Gew.-% eines Präpolymers mit aliphatischen Diisocyanat-Gruppen, 4 bis 20 Gew.-% Diamin auf Polyethylenoxidbasis, 10 bis 30 Gew.-% eines mehrwertigen Alkohols ausgewählt aus der Gruppe bestehend aus Propylenglycol und/ oder Glycerin und mindestens 30 Gew.-% Wasser gebildet wird.

Ganz besonders bevorzugt kann vorgesehen sein, dass die Hydrogelzusammensetzung aus 6 bis 20 Gew.-% Präpolymer mit Isophorondiisocyanat-Enden, 4 bis 15 Gew.-% Diamin auf Polyethylenoxidbasis, 15 bis 20 Gew.-% Polypropylenglycol und/ oder Glycerin und mindestens 40 Gew.-% Wasser gebildet wird.

Diese Hydrogelzusammensetzung weist üblicherweise eine freie Absorption A3 (gemessen nach DIN EN 13723-1 (2002)) von mindestens 1 g/g und höchstens 5 g/g auf, stellt ein nicht reizendes, flüssigkeitsabsorbierendes, vor Bakterien schützendes, polsterndes, hautartiges Medium bereit und ist somit besonders gut als Wundkontaktschicht geeignet.

Als besonders vorteilhafte Ausführungen haben sich weiterhin Wundauflagen gezeigt, die eine Hydrogelmatrix umfassen, deren Schichtdicke 0,1 bis 5,0 mm aufweist. Insbesondere weist damit eine erfindungsgemäße Wundauflage eine Wundkontaktschicht mit einer Schichtdicke von 0,1 bis 5,0 mm, insbesondere von 0,5 bis 5,0 mm und ganz besonders bevorzugt von 0,5 bis 3,0 mm auf. Wundauflagen mit solchen Schichtdicken zeigen einerseits keine Wundverklebung und andererseits die Fähigkeit, ein von einer Wunde abgegebenes Wundexsudat aufzunehmen und an die absorbierende Schicht weiterzuleiten. Diese Schichtdicken können an jeder Stelle der Wundkontaktschicht gleich sein oder in verschiedenen Bereichen der Wundkontaktschicht verschiedene Werte annehmen.

Weiterhin bevorzugt kann die Hydrogelmatrix Kanäle umfassen, insbesondere konische Kanäle, zum Durchtritt von Flüssigkeiten von der ersten zur zweiten Seite. Hierdurch kann insbesondere ein verbesserter Durchtritt für Wundexsudat bereitgestellt werden. Hierbei ist besonders bevorzugt vorgesehen, dass die Kanäle einen elliptischen oder einen kreisförmigen Querschnitt aufweisen, d.h. dass die Kanäle eine kreisförmige oder elliptische Öffnung sowohl an der der ersten als auch an der zweiten Seite der Hydrogelmatrix aufweisen, wobei die kreisförmige oder elliptische Öffnung auf der ersten und der zweiten Seite verschieden groß sind. Es kann jedoch auch vorgesehen sein, dass die Kanäle einen dreieckigen, rechteckigen, quadratischen, fünfeckigen, sechseckigen oder einen anderen vieleckigen Querschnitt aufweisen. Dabei ist ganz besonders bevorzugt vorgesehen, dass die erste Seite Öffnungen aufweist, die im Vergleich zu der auf der zweiten Seite befindliche Öffnung größer ist.

In einer weiteren bevorzugten Ausführungsform besteht die Hydrogelzusammensetzung zu 10 bis 25 Gew.-%, bevorzugt von 15 bis 20 Gew.-% aus einem festen polymeren Anteil und zu 75 bis 90 Gew.-%, bevorzugt 80 bis 85 Gew.-% aus einem flüssigen Anteil. Üblicherweise liegt hierbei der Anteil an gereinigtem Wasser in dem flüssigen Anteil in einem Bereich von 40 bis 90 Gew.-%, bevorzugt von 50 bis 80 Gew.-% und der Anteil an Glycerin in einem Bereich von 5 bis 40 Gew.-%, bevorzugt von 10 bis 30 Gew.-%.

Der polymere Anteil besitzt beispielsweise ein Gewichtsverhältnis von einem NCO-terminierten 3-armigen Poly(Ethylenoxid-*stat*-Propylenoxid) (Verhältnis PEO/PPO: 70:30 bzw. 80:20) zu einem Amin- terminiertem linearem Poly(Etylentylenoxid-co-Propylenoxid) von 1,0 bis 2,0, bevorzugt von 1,2 bis 1,8. Ein typisches Präpolymer mit aliphatischen Diisocyanat-Gruppen ist beispielsweise kommerziell erhältlich als Aquapol® PL-13000-3 (Carpenter; Richmond, USA), welches 4-7 Gew.-% Isophorondiisocyanate enthält.

Ein typisches Polyamin ist beispielsweise kommerziell erhältlich als Jeffamin® ED-2300, Huntsman; Everberg, Belgien, welches ein wasserlösliches aliphatisches Polyetheramin enthält, abgeleitet von Propylenoxid-gekapptem Polyethylenglycol. Die Hydrogelzusammensetzung ist insbesondere frei von Chloridionen, wie beispielsweise in einigen handelsüblichen Hydrogelen in Form isotonischer Kochsalzlösungen enthalten, um eine Ausfällung der Silberpartikel in Form von Silberchlorid und eine hierdurch bedingte Verringerung der Wirksamkeit zu vermeiden.

Ferner umfasst die Hydrogelzusammensetzung ein nichtionisches Tensid. Hierdurch soll insbesondere eine Stabilisierung wie eine homogene Verteilung der Silberpartikel in der Hydrogelzusammensetzung erreicht werden. Insbesondere kann eine hydrophobe Stabilisierung und Anbindung der Silberpartikel in die Polymerstruktur des Hydrogels mittels Stabilisatoren erfolgen, z.B. durch die Verwendung von Tensiden wie Tween 20, Polyethylenglycol, Sorbitol, Polyvinylpyrrolidon und Mischungen davon, insbesondere durch Tween 20 oder Sorbitol. In einer besonders vorteilhaften Ausführungsform umfasst die wasserhaltige Hydrogelzusammensetzung 6 bis 20 Gew.-% eines Präpolymers mit Isophorondiisocyanatenden, 4 bis 15 Gew.-% eines Diamins auf Polyethylenoxidbasis, 15 bis 20 Gew.-% Polypropylenglycol und/oder Glycerin, 40 bis 70 Gew.-% Wasser und 25 bis 250 ppm elementares Silber mit einem durchschnittlichen Partikeldurchmesser von 9-18 nm, alle Gewichtsangaben bezogen auf das Gesamtgewicht der Hydrogelzusammensetzung.

In einem weiteren Aspekt betrifft die vorliegende Erfindung ein Verfahren zur Herstellung einer Hydrogelzusammensetzung wie zuvor beschrieben, umfassend das Umsetzen einer Mischung, enthaltend:

(a) ein Polyamin,
(b) ein nichtionisches Tensid,
(c) optional einen mehrwertigen Alkohols ausgewählt aus Propylenglycol und/oder Glycerin,
(d) elementare Silberpartikel mit einem durchschnittlichen Partikeldurchmesser von 5-20 nm, bestimmt durch Transmissionselektronenmikroskopie (TEM), und einer Partikelgrößenverteilung, bestimmt durch Laserdiffraktometrie, von D90 ≤ 25 nm, und
(e) Wasser,

mit einem aliphatischen Diisocyanatpolymer, um eine wasserhaltige Hydrogelzusammensetzung zu bilden, welche ein Polyurethan-Polyharnstoff-Copolymer umfasst, wobei der Silbergehalt, bezogen auf das Gesamtgewicht der Hydrogelzusammensetzung, zwischen 15 und 500 ppm liegt.

Bezüglich bevorzugter Ausführungen der Komponenten (a) bis (e) wird auf die Erläuterungen zur Zusammensetzung des erfindungsgemäßen Hydrogels verwiesen, welche gleichermaßen für das erfindungsgemäße Verfahren Anwendung finden.

In einem weiteren Aspekt betrifft die vorliegende Erfindung eine mehrschichtige Wundauflage, umfassend mindestens eine wasserundurchlässige und wasserdampfdurchlässige Trägerschicht, eine absorbierende Schicht sowie eine die erfindungsgemäße wasserhaltige Hydrogelzusammensetzung umfassende Schicht.

Als Trägerschicht können insbesondere Polymerfilme oder Polymerschäume eingesetzt werden, vorzugsweise Filme oder Schäume, die aus Polyurethan, Polyetherurethan, Polyesterurethan, Polyether-Polyamid-Copolymeren, Polyacrylat oder Polymethacrylat gefertigt werden. Insbesondere ist als Trägerschicht ein wasserundurchlässiger und wasserdampfdurchlässiger Polyurethanfilm oder ein wasserundurchlässiger und wasserdampfdurchlässiger Polyurethanschaum geeignet. Insbesondere ist als Polymerfilm ein Polyurethanfilm, Polyesterurethanfilm oder Polyetherurethanfilm bevorzugt. Ganz besonders sind aber auch solche Polymerfilme bevorzugt, die eine Dicke von 15 $\mu$m bis 50 $\mu$m, insbesondere 20 $\mu$m bis 40 $\mu$m und ganz besonders bevorzugt von 25 $\mu$m bis 30 $\mu$m aufweisen. Die Wasserdampfdurchlässigkeit des Polymerfilms der Wundauflage weist vorzugsweise mindestens 750 g/m$^2$/24 Std., insbesondere mindestens 1000 g/m$^2$/24 Std. und ganz besonders bevorzugt mindestens 2000 g/m$^2$/24 Std. auf (gemessen nach DIN EN 13726). In besonders bevorzugten Ausführungsformen weisen diese Filme einen feuchtigkeitsdichten, klebenden Randabschnitt auf. Dieser Randabschnitt gewährleistet, dass das Wundsystem an seinem bestimmungsgemäßen Ort appliziert und fixiert werden kann. Darüber hinaus ist sichergestellt, dass keine Flüssigkeit zwischen der Folie und der die zu behandelnden Fläche umgebende Haut austreten kann. Als besonders bevorzugt sind solche Klebstoffe zu betrachten, die in einem dünnen Auftrag von 20 g/m$^2$ bis 35 g/m$^2$ zusammen mit dem Film eine Wasserdampfdurchlässigkeit von mindestens 800 g/m$^2$/24 Std. und vorzugsweise von mindestens 1000 g/m$^2$/24 Std. (gemessen nach DIN EN 13726) aufweisen.

In einer bevorzugten Ausführungsform umfasst die absorbierende Schicht einen hydrophilen Polyurethanschaum sowie das Hydrogel. Beispielsweise kann die Oberfläche eines hydrophilen Polyurethanschaums mit dem Hydrogel imprägniert bzw. beschichtet oder von diesem ganz oder teilweise durchsetzt sein.

In einer alternativen Ausführungsform kann die Hydrogelzusammensetzung auch benachbart oder räumlich getrennt von der absorbierenden Schicht vorliegen. Beispielsweise kann die Hydrogelzusammensetzung, umfassend ein Polyurethan-Polyharnstoff-Copolymer auf eine Oberfläche einer Schicht aus einem Polyurethanschaum beschichtet werden, so dass eine die Hydrogelzusammensetzung umfassende Hydrogelschicht in direktem Kontakt auf einer Schicht aus Polyurethanschaum aufliegt. Alternativ können die Hydrogelschicht und die absorbierende Schicht auch durch eine Abstandsschicht voneinander getrennt sein. Beispielsweise kann die Abstandschicht eine Hydrogelmatrix, ein Polymerfilm, eine Hydrokolloidmatrix, ein Polymernetz, ein Textilgewebe, ein Adhäsiv und/oder ein Polymernetz umfassen.

Ferner kann die mehrschichtige Wundauflage auch weitere Schichten neben der absorbierenden Schicht und der Trägerschicht umfassen, wie beispielsweise eine Wundkontaktschicht, eine oder mehrere Barriereschichten und/oder eine oder mehrere Verteilerschichten.

Bevorzugte Wundauflagen umfassen eine Trägerschicht, eine Hydrogelschicht gemäß der vorliegenden Erfindung und fakultativ eine zwischen der Hydrogelschicht und der Trägerschicht angeordnete absorbierende Schicht. Die absorbierende Schicht kann bevorzugt ein Fasermaterial, besonders bevorzugt einen hydrophilen Polyurethanschaum, umfassen. Die Hydrogelschicht kann kontinuierlich oder diskontinuierlich sein. Sie kann beispielsweise vollflächig auf die Trägerschicht aufgebracht sein oder Kanäle, Löcher oder anders geformte Öffnungen aufweisen. Bei einer diskontinuierlichen Hydrogelschicht kann eine Vielzahl diskreter Hydrogelelemente auf der Trägerschicht und/oder auf der absorbierenden Schicht aufgebracht sein, die die Form von Kreisen, Quadraten oder anderen regelmäßigen oder unregelmäßigen Vielecken aufweisen können. Mögliche Anordnungen der verschiedenen Schichten in erfindungsgemäßen mehrschichtigen Wundauflagen sind beispielsweise in der WO 2010/000450 beschrieben.

Die Wundauflagen weisen ferner einen hohen Komfort für den Patienten auf, indem sie einfach anzuwenden, hautfreundlich, weich, dünn, hautanpassend und analgetisch (über einen Hydrogel-Kühlungseffekt) sind und können somit auch über einen langen Zeitraum angewendet werden, üblicherweise 3 bis 5 Tage, bevor ein Wechsel der Wundauflage erfolgt.

Wie einleitend beschrieben kann der Heilungsprozess unabhängig von der Art der Wunde in unterschiedliche Phasen typisiert werden. Im Fachgebiet wird zwischen Inflammations-(Reinigungs-, Entzündungsphase), Granulations-(Proliferation) und Epithelisierungsphase unterschieden. Gemeinsames Merkmal ist, dass unterschiedliche Zelltypen miteinander interagieren, aktiviert werden und proliferieren.

Die Gefahr der Wundinfektion ist in der Inflammationsphase und Granulationsphase am höchsten, da hier besonders leicht bakterielle Erreger in die Wunde eindringen können. Die durch die erfindungsgemäße Wundauflage erzielten vorteilhaften Effekte sind somit in diesen Phasen besonders ausgeprägt.

Besonders wichtig ist die antibakterielle Wirkung bei langsam heilenden Wunden, wie beispielsweise Brandwunden oder chronischen Wunden. Die erfindungsgemäße Wundauflage ist somit zur Wundbehandlung von Brandwunden und/oder chronischen Wunden besonders geeignet, insbesondere von Wunden, wie sie bei Dekubitus, Ulcus cruris und diabetischem Syndrom auftreten.

## Beschreibung der Figuren

**Figur 1:** Freisetzung von Silbernanopartikeln aus dem Hydrogel ins wässrige Medium
**Figur 2:** Freisetzung von Silberionen aus dem Hydrogel ins wässrige Medium
**Figur 3A/B:** Antimikrobielle Aktivität des Hydrogels bei unterschiedlichem Silbergehalt
**Figur 4:** Zytotoxizität und Zellkompatibilität des Hydrogels bei unterschiedlichem Silbergehalt

**Figur 5A/B:** Absorptionskapazität eines erfindungsgemäßen Hydrogels bei unterschiedlichem Silbergehalt
**Figur 6A/B:** Dehydration eines erfindungsgemäßen Hydrogels bei unterschiedlichem Silbergehalt

**Beispiele**

Herstellung einer Hydrogelzusammensetzung

In einem ersten Ansatz wird eine Mischung aus 52,5 Gew.-% Polyamin (Jeffamin® ED-2003, Fa. Huntsman; Everberg, Belgien) und 47,5 Gew.-% Wasser gemischt. Von dieser Mischung werden 132,5g mit 200g Glycerin, 567,5g Wasser und der entsprechenden Menge einer wässrigen Silbernanopartikel-Suspension (agpure® W10, RAS materials GmbH, Regensburg, Deutschland) mit einem nominalen Silbergehalt von 10 Gew.%, einer Partikelgrößenverteilung von D99 <20 nm und einer durchschnittlichen Partikelgröße von 15 nm vermischt. Zu dieser Mischung werden 100 g eines IPDI-basierten Präpolymers (Aquapol® PI-13000-3; Fa. Carpenter; Richmond, USA) gegeben. Die Komponenten werden durchmischt, das noch flüssige Gel wird in Petrischalen portioniert und polymerisiert dort aus.

Jeffamin-Mix:

| | | |
|---|---|---|
| Jeffamin ED-2003 | Fa. Huntsman; Everberg, Belgien | 52,5 Gew.-% |
| Aqua purificata | Wasseraufbereitungsanlage | 47,5 Gew.-% |

Aquapol:

| | | |
|---|---|---|
| Aquapol PI-13000-3 | Fa. Carpenter; Richmond, USA | 100,0 Gew.-% |

Komponenten für 1 kg Hydrogel:

| Chemikalie | Einwaage [g] | Anteil Gel [%] |
|---|---|---|
| Glycerin | 200 | 20 |
| Wasser | 567,5 | 56,75 |
| Jeffamin-Mix | 132,5 | 13,25 |
| Aquapol | 100 | 10 |
| Silber | 0,0125 bis 0,25 | 0,00125 bis 0,025 (12,5 ppm bis 250 ppm) |

Messmethoden

**Nanopartikelfreisetzung**

Herstellung der Kalibrierkurve

Um die Migration der Silbernanopartikel aus dem Gel in das wässrige Medium zu untersuchen, wird das Eluat der Ionenfreisetzung mittels Photometer analysiert. Die Nanopartikel haben ein Absorptionsmaximum im sichtbaren Bereich bei ca. 410 nm. Um die Konzentration der Silbernanopartikel im Eluat bestimmen zu können, werden Standards in den Konzentrationen 50; 25; 10; 1; 0,1 mg Ag Pure® W 10/kg VE-Wasser gemessen, mit denen eine Kalibrierkurve erstellt werden kann.

Bestimmung der freigesetzten Silbernanopartikel

Für die Bestimmung der Ionenfreisetzung werden Prüfstücke mit einem Durchmesser von 50 mm ausgestanzt, von der Petrischale entfernt und gewogen. Die Gele werden in 100 mL Erlenmeyerkolben überführt, 30 mL Wasser (HPLG Grade) zugegeben und mit einem Schliffstopfen verschlossen. Danach werden die Proben für 24 Stunden und 130 rpm bei Raumtemperatur auf einem Schüttler inkubiert. Nach der Inkubation von 24 Stunden wird die Extinktion des Eluats bei 410 nm im Photometer gemessen. Es werden Polystyrolküvetten verwendet.

**Ionenfreisetzung**

Für die Bestimmung der Ionenfreisetzung werden Prüfstücke mit einem Durchmesser von 50 mm ausgestanzt, von der Petrischale entfernt und gewogen. Die Gele werden in 100 mL Erlenmeyerkolben überführt, 30 mL Wasser (HPLG Grade) zugegeben und mit einem Schliffstopfen verschlossen. Danach werden die Proben für 24 Stunden und 130 rpm bei Raumtemperatur auf einem Schüttler inkubiert. Anschließend werden ca. 5-6 mL der Eluate in braune Fläschchen pipettiert und zur Stabilisierung mit 20 µL konzentrierter Salpetersaure versetzt. Die Proben werden per IPC-MS gemäß EN ISO 17294-2 (E29) untersucht.

Freisetzungskinetik

Um die Freisetzung der Silbernanopartikel bzw. Silberionen über einen längere Zeitraum zu beobachten, werden die entsprechenden Messungen über die Prüfzeitpunkte 2; 4; 6; 8; 24; 48 und 72 Stunden bestimmt. Aus den AgNP-Gelen werden Prüfstücke mit dem Durchmesser von 50 mm ausgestanzt, von der Petrischale abgezogen und gewogen. Anschließend werden die Gele wie in den entsprechenden Abschnitten beschrieben behandelt.

**Wirksamkeitsstudien**

**Gebrauchskulturen und Medien**

Für die Hemmhof- und Weichagarmethode werden 24-Stunden-Kulturen vom Bakterienstamm *Staphylococcus aureus* DSM 346 verwendet. Dafür wird eine Kryokugel in 9 mL Caso-Boullion überführt und 24 Stunden bei 37 °C im Brutschrank inkubiert. Die Suspension wird visuell auf eine starke Trübung und somit gutes Wachstum hin begutachtet.

**Plattengussverfahren**

Die Auswertung der lebensfähigen Bakterien erfolgt über das Auszählen der Kolonie-bildenden Einheiten (KBE). Eine definierte Menge Bakteriensuspension wird in warmen CaSo-Agar gemischt, und nach der Inkubation bei der geeigneten Temperatur ausgezählt. Man geht davon aus, dass jede Bakterienzelle dabei eine Kolonie auf der Platte bildet.
Beim Plattengussverfahren werden von den Proben geeignete Verdünnungsstufen in 10er-Schritten (1:10) hergestellt und davon jeweils 1 mL in eine leere Petrischale gegeben. Danach werden ca. 20 mL auf 45 °C temperierter CaSo-Agar in die Petrischale gegossen und die Platte in einer 8-förmigen Bewegung geschwenkt, damit sich die Bakterienzellen gleichmäßig im Agar verteilen. Die Platten werden bei Raumtemperatur gelagert, bis sie erstarrt sind. Zuletzt werden sie bei 37 °C im Brutschrank ca. 24 Stunden inkubiert und die gebildeten Bakterienkolonien anschließend ausgezählt. Für die Auswertung werden die KBE/mL berechnet.

KBE: ausgezählte Kolonien
Vf Weichagar: Verdünnungsfaktor Weichagar
Vf D/e-Enthemmer: Verdünnungsfaktor Weichagar
Vf Plattenguss: Verdünnungsfaktor der ausgezählten Platten

$$KBE\ /mL = ausgezählte\ KBE * Vf\,Weichagar * Vf\,D/E{-}Enthemmer * Vf\,Plattenguss$$

**Hemmhoftest**

Um einen ersten Eindruck von der antimikrobiellen Wirksamkeit der AgNP-Gele zu bekommen, werden die Proben auf frisch beimpfte Agarplatten gelegt und ausgewertet, ob die Proben das Wachstum der Bakterien hemmen. Für die Hemmhofmethode werden kreisrunde Prüfstücke mit dem Durchmesser von 30 mm ausgestanzt. Auf fertige CaSo-Platten werden 5 mL einer Bakteriensuspension, 1 mL suspendiert in 150 mL flüssigem Caso-Agar, pipettiert und gewartet, bis die Platten ausgehärtet sind. Die Platten müssen innerhalb einer Stunde verwendet werden, damit das Bakterienwachstum nicht vor dem Auflegen der Prüflinge beginnt. Die Prüfstücke werden mit der aktiven Seite zur Agarplatte hin aufgelegt und angedrückt, damit die Probe guten Kontakt zur Platte hat , und bei 37°C über Nacht im Brutschrank inkubiert. Anschließend wird der gebildete Hemmhof ausgemessen.

$$H=(D-d)/2$$

H = Hemmzone [mm]
D = Gesamtdurchmesser [mm]
d = Durchmesser der Probe [mm]

**Weichagartest**

Um genauere Ergebnisse als beim Hemmhoftest zu bekommen, wird eine definierte Konzentration von Bakterien in Weichagar auf die zu prüfenden Proben aufgegeben und die KBE/mL nach einer Prüfzeit von vier Stunden ausgewertet.

100 mL Weichagar werden auf 45 °C temperiert und mit 1 mL der Bakteriensuspension beimpft. Es werden Probenstücke der Größe 2,5 cm x 2,5 cm mit einem Stanzeisen ausgestanzt und einer sterilen Pinzette in leere Petrischalen überführt. Je 1 mL des beimpften Weichagars wird mit einer Pipette auf die Oberfläche der Probenstücke aufgetragen und darauf geachtet, dass der Weichagar nicht herunter fließt. Dieser erstarrt bei Raumtemperatur nach ca. 10 Minuten. Zusätzlich wird eine Positivkontrolle angesetzt. Hierfür wird 1 mL beimpfter Weichagar in ein 50 mL Falcon-Tube pipettiert und 3 mL 1/4 starke Ringerlösung zugegeben, damit der Weichagar nicht austrocknet. Die beimpften Probenstücke werden bei 37 °C im Brutschrank über Nacht inkubiert. Nach der Prüfzeit von vier Stunden werden 20 mL Dey-Engley-Enthemmer dazu gegeben, um die noch frei vorhandenen Silberionen zu binden. Für den 0-Stunden-Wert wird 1 mL des beimpften Weichagars direkt in die 20 mL D/E-Enthemmer pipettiert.

Die Ansätze werden eine Minute im Ultraschallbad behandelt. Anschließend werden geeignete Verdünnungsreihen mit 1/4 starker Ringerlösung hergestellt und mit dem Plattengussverfahren ausplattiert.

$$\text{Geometrisches Mittel der Probe} = (\log x_1 + \log x_2 + \log x_3)/(3)$$

$$\text{Geometrisches Mittel der Kontrolle} = (\log xk_1 + \log xk_2 + \log xk_3)/(3)$$

$$\text{Log-Stufen Reduktion} = \text{Log-Stufe Kontrolle} - \text{Log-Stufe Probe}$$

**Messung des Feuchtigkeitsverlusts (Dehydration)**

Als Feuchtigkeitsverlust wird der Verlust an Gewicht über einen bestimmten Zeitraum bei einer definierten Temperatur beschrieben. Der Feuchtigkeitsverlust berechnet sich nach folgender Gleichung und wird in der Einheit g/g angegeben:

$$\text{Feuchtigkeitsverlust} = \text{Endgewicht} / \text{Anfangsgewicht}$$

**Messung der Absorptionskapazität**

Zur Messung der Absorptionskapazität werden Gelproben mit Durchmesser 5 cm ausgestanzt. Anschließend werden sie in ein Becherglas mit V = 300 ml deionisiertem Wasser gegeben. Daraufhin werden sie in bestimmten Zeitabständen erneut gewogen. Die Berechnung der Absorptionskapazität erfolgt nach folgender Gleichung und wird in der Einheit g/g angegeben:

$$\text{Absorptionskapazität} = (\text{Endgewicht} - \text{Anfangsgewicht}) / \text{Anfangsgewicht}$$

**Test auf Zellkompatibilität**

Die Tests auf Zellkompatibilität wurden gemäß DIN EN ISO 10993-5 und den Verfahrensanweisungen der Abteilung für Funktionswerkstoffe der Medizin und der Zahnheilkunde: BioLab 973302, 042901, 964702 und 964805 durchgeführt und umfassen Messungen des Zellwachstums, der Stoffwechselaktivität und des Proteingehalts.

Die Hydrogele wurden steril in Petrischalen geliefert. Zur Prüfung wurden je 0,1 g/ml Kulturmedium der Proben eingewogen.

Die Zellaktivität, die Zellzahl sowie die Proteinkonzentration wurden pro Probe dreimal in je vier Parallelansätzen untersucht. Die Eluationszeit betrug 48 h, die Inkubation der Zellen mit den Eluaten ebenso 48 h.

| | |
|---|---|
| Zelllinie: | L 929 CC1 Mausfibroblasten (American Type Culture Collection, Rockeville Maryland, USA). |
| Kulturmedium: | DMEM (Dulbecco's mod. Eagle Medium) nach VA BioLab 042901 zur Vorkultur und Eluation. |
| Negativkontrolle: | Polystyrol (Firma Nunc GmbH & Co KG, Wiesbaden). |
| Positivkontrolle: | Vekoplan KT PVC Platten (König GmbH, Wendelstein). |

Pro Probe wurden drei Eluate aus je einem Hydrogel getestet, die an unterschiedlichen Versuchstagen angesetzt wurden. Hierzu wurden die Hydrogele in den Petrischalen mit einem sterilen Skalpell mittig halbiert und in ein steriles 50 ml Reaktionsgefäß überführt. Pro 0,1 g Probe wurde 1 ml Eluationsmedium zu den Hydrogelen zugegeben und diese dann für 48 h bei 37 °C und 5 % $CO_2$ im Inkubator eluiert. Um vorhandene Schwebstoffe aus den Eluaten zu entfernen, wurden die Proben nach der Inkubation für 5 min bei 4000 rpm zentrifugiert und durch einen Filter (Porengröße 0,2 $\mu$m) filtriert.

Die Zellen wurden in der Konzentration 50.000 Zellen/ml ausgesät, die Vorkultivierung erfolgte bei 37 °C und 5 % $CO_2$ für 24 h. Anschließend wurde das bei der Aussaat zugegebene DMEM Medium abgezogen und die Zellen mit je 1 ml Eluat in der Konzentration 100 % bedeckt. Als Negativkontrolle wurde DMEM Medium 48 h in einem 50 ml Falconröhrchen wie die Proben inkubiert, als Positivkontrolle diente das Eluat der Plastic Discs in der Konzentration 100 %. Nach 48 stündiger Inkubation wurden die Zellaktivität, die Zellzahl und der Gesamtproteingehalt bestimmt.

### Zellwachstum (Zellzählung)

Die Zellzählung erfolgte nach enzymatischer Ablösung der Zellen mittels Accutase mit Hilfe des Zellzählers.

### Vitalitätstest über Stoffwechselaktivität (WST)

Die Prüfung der Vitalität erfolgt mit Tetrazoliumsalz, WST 1, Fa. Roche Diagnostics GmbH Mannheim, nach Angabe des Herstellers. WST 1 wird durch die Succinatdehydrogenase (Enzym des Citronensäurezyklus) in den Mitochondrien der stoffwechselaktiven Zellen zum farbigen Formazan umgesetzt und photometrisch vermessen. Die Absorptionswerte (OD), bei 450 nm und 620 nm bestimmt, korrelieren mit der Atmungsaktivität der kultivierten Zellen.

### Proteingehalt (Lowry)

Die Prüfung des Proteingehaltes erfolgt mit dem DC Protein Assay, Fa. BIO-RAD GmbH München, nach Angabe des Herstellers. Die Proteinbestimmung nach Lowry basiert auf der Reduktion von Cu(II) zu Cu(I) durch die aromatischen Tyrosin-Tryptophanreste von Proteinen. Der Kupfer-Protein-Komplex reduziert in einem weiteren Schritt ein Phosphormolybdänsäure-Phosphorwolframat-Reagenz zu Molybdän bzw. Wolframblau. Die Extinktion dieser intensiven blauen Färbung wird photometrisch bei 750 nm gemessen. Durch das Mitführen einer Standardreihe kann die Proteinkonzentration bestimmt werden.

### Akzeptanz und Auswertung

Die Einteilung der Wertungsbereiche für Akzeptanz und Auswertung erfolgte in Anlehnung an DIN EN ISO 7405 und dem Begriff Inhibitionsdosis (ID 50: Dosis bei welcher 50% der Zellen im Wachstum gehemmt sind) (Literatur: Allgemeine Pharmakologie und Toxikologie, Henschler, Hrsg.: Forth Wolfgang; Spektrum akad. Verl. Heidelberg; 7. Aufl. 1996). Als starke Wachstumshemmung wird ein Zellwachstum von 0-29 %, als mäßige Hemmung ein Zellwachstum von 30-59 % und als schwache Hemmung ein Zellwachstum von 60-79 % im Vergleich zur Kontrolle charakterisiert. Zellwachstumsraten zwischen 80 und 100 % indizieren ein ungehemmtes Zellwachstum.

Als stark verminderte Stoffwechselaktivität wird eine Zellaktivität von 0-29 %, als mäßig verminderte Stoffwechselaktivität eine Zellaktivität von 30-59 % und als schwach verminderte Stoffwechselaktivität eine Zellaktivität von 60-79 % im Vergleich zur Kontrolle charakterisiert. Zellaktivitätsraten zwischen 80 und 100% indizieren eine nicht verminderte Stoffwechselaktivität.

Als stark reduzierter Proteingehalt wird eine Proteinkonzentration von 0-34 %, als mäßig reduzierter Proteingehalt eine Proteinkonzentration von 35-69 % im Vergleich zur Kontrolle charakterisiert. Proteinkonzentrationen zwischen 70 und 100 % indizieren einen nicht reduzierten Proteingehalt.

Der PS-Wert in der Ergebnisdarstellung entspricht der Polystyrol-Negativkontrolle.

**Beispiel 1:** Freisetzung von Silberpartikel und Silberionen aus dem Gel ins wässrige Medium

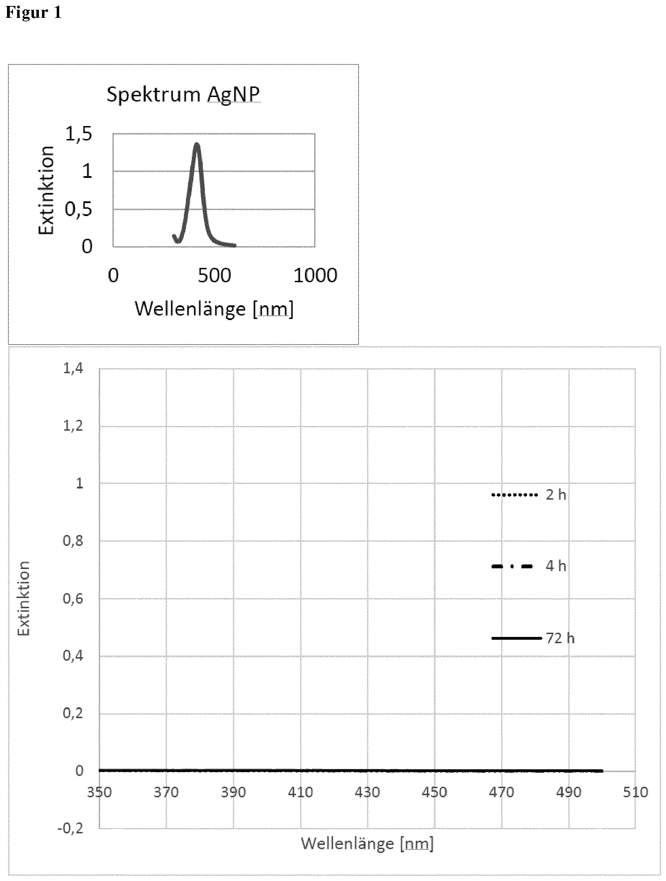

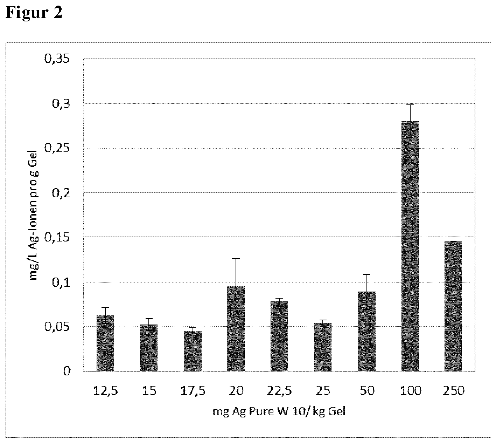

Durch die Bestimmung von Freisetzungskinetiken wurde die Migration von Silbernanopartikel sowie Silberionen aus einem wie zuvor beschrieben hergestellten Hydrogel in das wässrige Medium untersucht. Es konnte gezeigt werden, dass keine nachweisbaren Mengen von Silberpartikeln ins wässrige Medium freigesetzt wurden (Figur 1). Die freigesetzte Menge an Silberionen lag in Abhängigkeit vom Silbergehalt bei 50 bis 300 $\mu$g/L Ag+ pro 1g Hydrogel, wie in Figur 2 gezeigt. In simulierter Wundflüssigkeit konnte eine Freisetzung von Silberionen im Bereich bis 250 mg/kg Hydrogel (250 ppm) gezeigt werden.

**Beispiel 2:** Antimikrobielle Aktivität des Hydrogels bei unterschiedlichem Silbergehalt

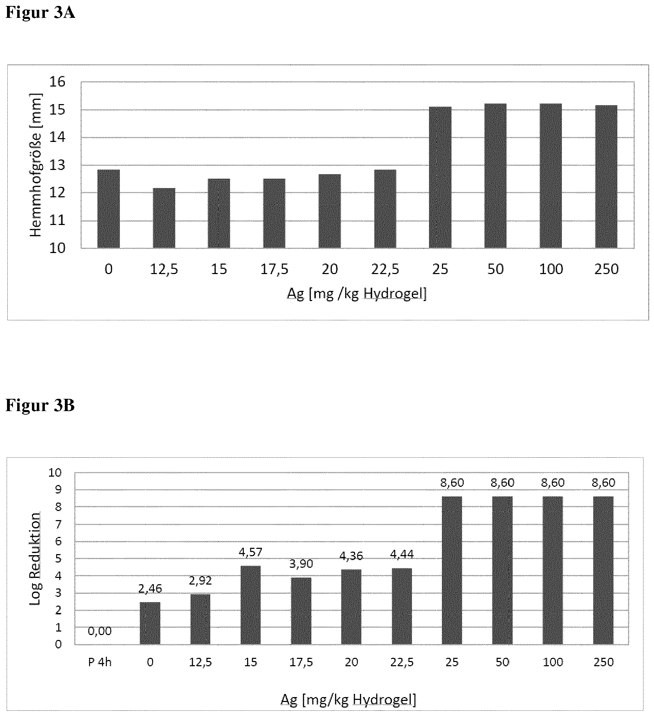

In Hemmhoftests konnte eine signifikante Hemmung des Wachstums von *Staphylococcus aureus* bereits ab einem Silbergehalt von 25 mg/kg Hydrogel beobachtet werden (Figur 3A. Die Hemmung erzielte im Bereich von 25 bis 250 mg/kg Hydrogel 8 log-Stufen (Figur 3B).

**Beispiel 3:** Zytotoxizität und Zellkompatibilität des Hydrogels bei unterschiedlichem Silbergehalt

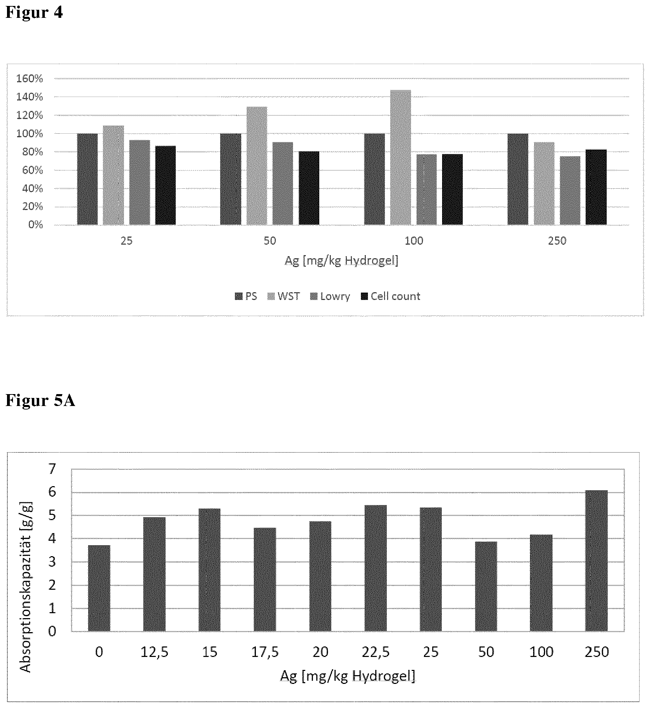

Die Zytotoxizität und Zellkompatibilität des Hydrogels bei unterschiedlichem Silbergehalt wurde durch Bestimmung der Zellaktivität, der Zellzahl sowie der Proteinkonzentration sowie durch einen Vitalitätstest der Stoffwechselaktivität (WST) ermittelt. In sämtlichen Tests konnte eine gute Zellkompatibilität bei geringer Zytotoxizität ermittelt werden (Figur 4).

**Beispiel 4:** Absorptionskapazität eines erfindungsgemäßen Hydrogels bei unterschiedlichem Silbergehalt

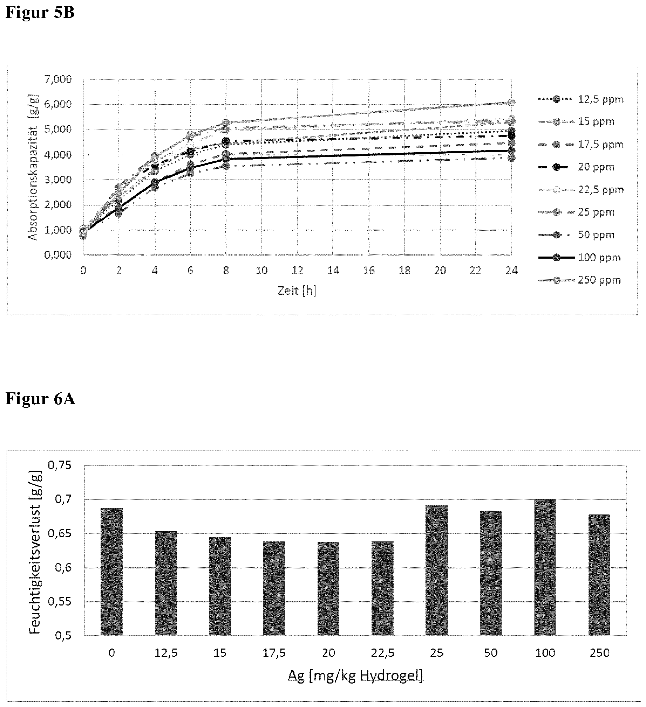

Es konnte durch Messung der Absorptionskapazität bei einem Zeitraum bis 24h eines erfindungsgemäßen Hydrogels bei unterschiedlichem Silbergehalt keine Änderung der Absorptionskapazität in Abhängigkeit des Silbergehalts außerhalb von statistischen Schwankungen gefunden werden (Figuren 5A und 5B).

**Beispiel 5:** Dehydration eines erfindungsgemäßen Hydrogels bei unterschiedlichem Silbergehalt

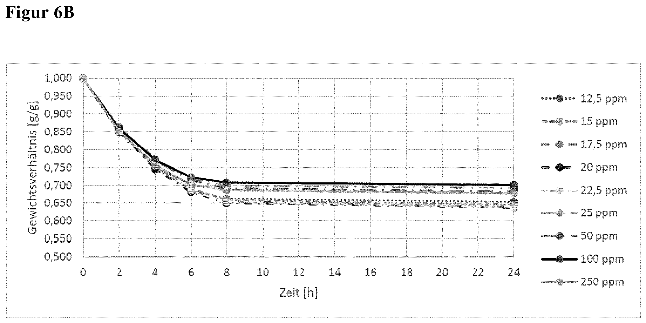

Es konnte durch Messung der Dehydration eines erfindungsgemäßen Hydrogels über einen Zeitraum von 24h bei unterschiedlichem Silbergehalt keine Änderung der Dehydration in Abhängigkeit des Silbergehalts außerhalb von statistischen Schwankungen gefunden werden (Figuren 6A und 6B).

Es konnte somit durch die gezeigten Beispiele überraschend gezeigt werden, dass durch Bereitstellung einer erfindungsgemäßen Hydrogelzusammensetzung eine ausreichend hohe Silberionenfreisetzung ermöglicht wird, um während der Wundbehandlung eine effektive antimikrobielle Aktivität zu gewährleisten. Gleichzeitig wird dabei jedoch die Freisetzung von Silberpartikeln und somit deren Aufnahme in den menschlichen Körper wirksam verhindert, so dass hierdurch bedingte Nebenwirkungen wirksam vermieden bzw. verhindert werden können.

Ferner konnte gezeigt werden, dass die im Hydrogel enthaltenden Silberpartikel keinen Einfluss auf dessen Absorptionskapazität oder Dehydration aufweisen, und somit die Funktion einer das Hydrogel enthaltenden Wundauflage nicht beeinträchtigen.

Es konnte somit durch Bereitstellung der erfindungsgemäßen Hydrogelzusammensetzung ein System zur Wundtherapie zur Verfügung gestellt werden, mit dem eine Wundbehandlung möglichst wirksam durchgeführt werden kann und welches eine hohe antimikrobielle Aktivität aufweist. Hierdurch kann die Wundheilung in effektiver Weise gefördert und beschleunigt werden. Die Hydrogelzusammensetzung enthaltenen Wundtherapiesysteme weisen einen hohen Tragekomfort auf und verfügen selbst bei langer Tragedauer über eine Effektive antimikrobielle Wirksamkeit.

**Patentansprüche**

1. Wasserhaltige Hydrogelzusammensetzung, umfassend elementare Silberpartikel mit einem durchschnittlichen Partikeldurchmesser von 5-20 nm, bestimmt durch Transmissionselektronenmikroskopie (TEM), und einer Partikelgrößenverteilung von D90 $\leq$ 25 nm, bestimmt durch Laserdiffraktometrie, wobei der Silbergehalt, bezogen auf das Gesamtgewicht der Hydrogelzusammensetzung, zwischen 15 und 500 ppm liegt, wobei die Hydrogelzusammensetzung ferner ein nichtionisches Tensid umfasst, wobei die Hydrogelzusammensetzung ein Polyurethan-Polyharnstoff-Copolymer umfasst.

2. Wasserhaltige Hydrogelzusammensetzung nach Anspruch 1, wobei der durchschnittliche Partikeldurchmesser von 9-18 nm beträgt.

3. Wasserhaltige Hydrogelzusammensetzung nach Anspruch 1 oder 2, wobei der Silbergehalt, bezogen auf das Gesamtgewicht der Hydrogelzusammensetzung, zwischen 25 und 250 ppm liegt.

4. Wasserhaltige Hydrogelzusammensetzung nach einem der vorhergehenden Ansprüche, wobei die Hydrogelzusammensetzung einen hydrophilen Polyurethanschaum umfasst.

5. Wasserhaltige Hydrogelzusammensetzung nach einem der vorhergehenden Ansprüche, wobei das Tensid ausgewählt ist aus Tween 20, Polyethylenglycol, Sorbitol und Polyvinylpyrrolidon, oder Mischungen davon.

6. Wasserhaltige Hydrogelzusammensetzung nach einem der vorhergehenden Ansprüche, ferner umfassend 5 bis 30 Gew.-%, bezogen auf das Gesamtgewicht der Hydrogelzusammensetzung, eines mehrwertigen Alkohols ausgewählt aus Propylenglycol und/oder Glycerin.

7. Mehrschichtige Wundauflage, umfassend mindestens eine wasserundurchlässige und wasserdampfdurchlässige Trägerschicht, eine absorbierende Schicht sowie eine die wasserhaltige Hydrogelzusammensetzung nach einem der Ansprüche 1 bis 6 umfassende Schicht.

8. Mehrschichtige Wundauflage nach Anspruch 7, wobei die absorbierende Schicht einen hydrophilen Polyurethanschaum sowie das Hydrogel umfasst.

9. Mehrschichtige Wundauflage nach Anspruch 8, wobei die Oberfläche des hydrophilen Polyurethanschaums zumindest partiell mit dem Hydrogel imprägniert ist.

10. Mehrschichtige Wundauflage nach einem der Ansprüche 7 bis 9, wobei die Wundauflage in der Inflammationsphase und/oder der Granulationsphase der Wundheilung eingesetzt wird.

11. Mehrschichtige Wundauflage nach einem der Ansprüche 7 bis 9 zur Verwendung in der Behandlung von Brandwunden und/oder chronischen Wunden.

12. Verfahren zur Herstellung einer Hydrogelzusammensetzung, umfassend das Umsetzen einer Mischung, enthaltend:

    (a) ein Polyamin,
    (b) ein nichtionisches Tensid,
    (c) optional einen mehrwertigen Alkohol ausgewählt aus Propylenglycol und/oder Glycerin,
    (d) elementare Silberpartikel mit einem durchschnittlichen Partikeldurchmesser von 5-20 nm, bestimmt durch Transmissionselektronenmikroskopie (TEM), und einer Partikelgrößenverteilung von D90 ≤ 25 nm, bestimmt durch Laserdiffraktometrie, und
    (e) Wasser,

mit einem aliphatischen Diisocyanatpolymer, um eine wasserhaltige Hydrogelzusammensetzung zu bilden, welche ein Polyurethan-Polyharnstoff-Copolymer umfasst, wobei der Silbergehalt, bezogen auf das Gesamtgewicht der Hydrogelzusammensetzung, zwischen 15 und 500 ppm liegt.

**Claims**

1. Water-containing hydrogel composition comprising elemental silver particles having an average particle diameter of 5-20 nm as determined by transmission electron microscopy (TEM) and a particle size distribution of D90 ≤ 25 nm as determined by laser diffraction, the silver content being between 15 and 500 ppm based on the total weight of the hydrogel composition, the hydrogel composition further comprising a non-ionic surfactant, wherein the hydrogel composition comprises a polyurethane-polyurea copolymer.

2. Water-containing hydrogel composition of claim 1, wherein the average particle diameter is from 9-18 nm.

3. Water-containing hydrogel composition according to claim 1 or 2, wherein the silver content, based on the total weight of the hydrogel composition, is between 25 and 250 ppm.

4. Water-containing hydrogel composition according to any one of the preceding claims, wherein the hydrogel composition comprises a hydrophilic polyurethane foam.

5. Water-containing hydrogel composition according to any one of the preceding claims, wherein the surfactant is selected from Tween 20, polyethylene glycol, sorbitol and polyvinyl-pyrrolidone, or mixtures thereof.

6. Water-containing hydrogel composition according to any one of the preceding claims, further comprising from 5 to 30% by weight, based on the total weight of the hydrogel composition, of a polyhydric alcohol selected from propylene glycol and/or glycerol.

7. Multilayer wound dressing comprising at least a water-impermeable and water vapour permeable carrier layer, an absorbent layer, and a layer comprising the water-containing hydrogel composition according to any one of claims 1 to 6.

8. Multilayer wound dressing according to claim 7, wherein the absorbent layer comprises a hydrophilic polyurethane foam and the hydrogel.

9. Multilayer wound dressing according to claim 8, wherein the surface of the hydrophilic polyurethane foam is at least partially impregnated with the hydrogel.

10. Multilayer wound dressing according to any one of claims 7 to 9, wherein the wound dressing is used in the inflammation phase and/or the granulation phase of wound healing.

11. Multilayer wound dressing according to any one of claims 7 to 9 for use in the treatment of burn wounds and/or chronic wounds.

12. Process for preparing a hydrogel composition comprising transposition of a mixture, containing:

(a) a polyamine,
(b) a non-ionic surfactant,
(c) optionally a polyhydric alcohol selected from propylene glycol and/or glycerol,
(d) elemental silver particles having an average particle diameter of 5-20 nm as determined by transmission electron microscopy (TEM) and a particle size distribution of $D90 \leq 25$ nm as determined by laser diffraction; and
(e) water,

with an aliphatic diisocyanate polymer to form a water-containing hydrogel composition comprising a polyurethane-polyurea copolymer, wherein the silver content is between 15 and 500 ppm based on the total weight of the hydrogel composition.

**Revendications**

1. Composition aqueuse d'hydrogel comprenant des particules élémentaires d'argent ayant un diamètre moyen de particule de 5-20 nm, déterminé par microscopie électronique à transmission (MET), et une distribution de taille de particule de $D90 \leq 25$ nm, déterminée par diffractométrie laser, la teneur en argent étant comprise entre 15 et 500 ppm par rapport au poids total de la composition d'hydrogel, la composition d'hydrogel comprenant en outre un agent tensioactif non ionique, la composition d'hydrogel comprenant un copolymère de polyuréthane-polyurée.

2. Composition aqueuse d'hydrogel selon la revendication 1, dans laquelle le diamètre moyen des particules est de 9 à 18 nm.

3. Composition aqueuse d'hydrogel selon la revendication 1 ou 2, dans laquelle la teneur en argent, par rapport au poids total de la composition d'hydrogel, est comprise entre 25 et 250 ppm.

4. Composition aqueuse d'hydrogel selon l'une quelconque des revendications précédentes, dans laquelle la composition d'hydrogel comprend une mousse de polyuréthane hydrophile.

5. Composition aqueuse d'hydrogel selon l'une quelconque des revendications précédentes, dans laquelle le tensioactif est choisi parmi le tween 20, le polyéthylène glycol, le sorbitol et la polyvinyl-pyrrolidone, ou mélanges de ceux-ci.

6. Composition aqueuse d'hydrogelselon l'une quelconque des revendications précédentes, comprenant en outre de 5 à 30 % en poids, par rapport au poids total de la composition d'hydrogel, d'un polyalcool choisi parmi le propylèneglycol et/ou le glycérol.

7. Pansement multicouche comprenant au moins une couche de support imperméable à l'eau et perméable à la vapeur d'eau, une couche absorbante et une couche comprenant la composition d'hydrogel contenant de l'eau selon l'une quelconque des revendications 1 à 6.

8. Pansement multicouche selon la revendication 7, dans lequel la couche absorbante comprend une mousse de polyuréthane hydrophile et l'hydrogel.

9. Pansement multicouche selon la revendication 8, dans lequel la surface de la mousse de polyuréthane hydrophile est au moins partiellement imprégnée de l'hydrogel.

10. Pansement multicouche selon l'une des revendications 7 à 9, dans lequel le pansement est utilisé dans la phase d'inflammation et/ou la phase de granulation de la cicatrisation.

11. Pansement multicouche selon l'une quelconque des revendications 7 à 9, destiné à être utilisé dans le traitement des brûlures et/ou des plaies chroniques.

12. Procédé de préparation d'une composition d'hydrogel, comprenant la réaction d'un mélange contenant :

> (a) une polyamine,
> (b) un agent tensioactif non ionique,
> (c) éventuellement un polyalcool choisi parmi le propylène glycol et/ou le glycérol,
> (d) des particules élémentaires d'argent ayant un diamètre moyen de particule de 5 à 20 nm, déterminé par microscopie électronique à transmission (MET), et une distribution granulométrique de $D90 \leq 25$ nm, déterminée par diffraction laser, et
> (e) de l'eau,

avec un polymère de diisocyanate aliphatique pour former une composition aqueuse d'hydrogel comprenant un copolymère de polyuréthane et de polyurée, dans laquelle la teneur en argent est comprise entre 15 et 500 ppm par rapport au poids total de la composition d'hydrogel.

**Figur 1**

**Figur 2**

**Figur 3A**

**Figur 3B**

## Figur 4

## Figur 5A

**Figur 5B**

**Figur 6A**

**Figur 6B**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2010000451 A
- WO 2011141454 A
- WO 2010000450 A

**In der Beschreibung aufgeführte Nicht-Patentliteratur**

- beschreiben antimikrobielle Carboxymethylcellulose-Hydrogele die nanopartikuläres Silber enthalten. **ANUP et al.** CARBOHYDRATE POLYMERS. APPLIED SCIENCE PUBLISHERS, LTD, 07. April 2015, 254-261
- Allgemeine Pharmakologie und Toxikologie. Spektrum akad. Verl, 1996